# EUROPEAN PATENT SPECIFICATION

(11) **EP 2 441 710 B1**
(45) Date of publication and mention of the grant of the patent: **30.01.2013**
(21) Application number: 11184783.6
(22) Date of filing: 12.10.2011
(51) Int. Cl.: B65G 47/90, A61M 5/00, B65B 5/08

(54) **A method and an apparatus for transferring articles from a packaging machine to containers**
Verfahren und Vorrichtung zur Abführung von Artikeln von der Verpackungsmachine zu Behältern
Méthode et appareil pour transférer des articles d'une machine d'emballage vers des conteneurs

(30) Priority: 14.10.2010 IT BO20100616
(43) Date of publication of application: 18.04.2012
(73) Proprietor: Marchesini Group S.p.A., 40065 Pianoro (Bologna) (IT)
(72) Inventor: Monti, Giuseppe, 40065 PIANORO (BOLOGNA) (IT)
(74) Representative: Dall'Olio, Christian

(56) References cited:
- EP-A1- 2 090 324
- EP-A1- 2 196 395
- DE-A1- 4 102 618
- DE-A1- 19 604 100
- DE-U1- 20 108 401
- DE-U1- 29 907 459
- DE-U1- 29 923 418
- GB-A- 2 290 221
- US-A- 3 133 635
- US-A- 5 806 287
- US-A1- 2003 009 994
- US-A1- 2006 191 240

## Description

The present invention relates to transfer methods and apparatus for picking up articles made of a fragile material in outlet from a packaging machine, and transferring the articles to protective and transport containers.

In particular, the invention relates to methods and apparatus used for transferring syringes with in glass tube "barrels" or small containers made of a fragile material and is particularly designed to contain liquids or other loose substances.

In the following, for ease of description, reference will be made purely by way of example to the problem of transfer of syringes with glass barrels for injections, in outlet from a packaging machine, which, for example, receives in inlet syringe barrels (receptacles for the liquid to be injected, an interior of which is connected to the channel of the needle) and processes them such as to pack the single syringes. The packaging machine can be, for example, a piston-inserting machine (for installing the plunger rod in the syringe and/or the needle) and/or a labelling machine.

The filled syringes, comprising the rod and with a lower safety cap in which the needle is embedded, are presented at the outlet of a packaging machine, arranged in a predefined formation, for example, in a single line.

The syringes must be collected at the output of the packaging machine and placed in containers for transport thereof, for example, to packing areas.

In various devices known in the field, a plurality of syringes to be transferred are simultaneously collected by the machine, to be housed a number at a time in the containers.

This is because it is clear that collecting a single syringe at a time would be detrimental to production performance and would thus cause a so-called "bottleneck" in the production chain of receiving, packaging and packing of syringes.

The devices currently known, during the collecting operations of syringes taken from the output of the machine, are made in such a way as to group the syringes in order then to place them, in various ways, into relative housings in the transport container.

During this stage, the syringes are quite possibly subject to impacting, comprising against one another.

Since the syringe barrels are made of a fragile material, impacts are often the cause of cracks and/or chipping, if not shattering, which leads to high costs and low yields.

Document EP 2.196.395 discloses a method for packaging nested syringes according to a prior art solution. This document also discloses an apparatus according to the preamble of claim 5.

The aim of the present invention is to obviate the above-described drawbacks, and others besides, with:
a method as in claim 1, for transferring a plurality of articles, at least partially made of a fragile material, for example syringes for injections, from an automatic machine which presents them at an outlet thereof separated from one another, to containers comprising a plurality of housings in each of which the articles are singly housable separately from one another; and
an apparatus that implements the method, in accordance with claim 5.

The method comprises following steps:
a. arranging a plurality of articles presented at the outlet of the automatic machine in a predefined formation in which they are maintained separated from one another;
b. removing more than one of the articles arranged in step a. and maintaining them separated from one another, taking them to a container; and c. inserting the articles, maintaining them separated from one another, into the container.

The apparatus comprises:
at least a drawing device for receiving and taking delivery of a plurality of articles which exit the machine as and when they are presented at the outlet thereof; and
at least a pick-up and place device for removing the articles from the drawing device, taking them to the container and inserting them in respective housings thereof.

Further, as stated in claim 7, in the apparatus according to the invention the following is comprised:
the drawing device comprises a plurality of mobile seatings for singly housing respective articles presented at the outlet of the machine and moves the housed articles distancingly from the outlet of the machine, the mobile seatings being arranged with respect to one another in such a way that the articles housed therein are maintained separated from one another, and wherein
the pick-up and place device comprises at least a gripping means configured such as to be able to pick up or alternatively release a plurality of articles and to maintain the picked-up articles separate from one another.

In the following, we will continue to refer to the particular case in which the articles comprise syringes for injections (i.e. substantially syringe barrels with possibly some accessories), which comprise syringe bodies made of a fragile material, but it is clear that this choice, except where it will be differently specified, has purely descriptive purposes and does not limit the scope of application of the invention.

In syringe packaging machines the syringes are packed at the output thereof separate from one another.

At the output, therefore, the syringes are not subject to reciprocal impact.

By using the method and/or the apparatus of the invention, the syringes exiting the machine are arranged such as to be immediately separated from one another.

The expression 'separated' in this context clearly means physically separated, i.e. not in contact and in any case sufficiently spaced such that normal vibrations and oscillations occurring during the movements caused by devices and machines do not cause any reciprocal collisions.

After which, the syringes are moved towards the container destined to house them still separated from one another, as stated in claims 1 and 5 as mentioned above, and therefore mutual collisions are impossible.

At this point, the syringes are inserted in their housings, and even during this operation are constantly kept separated.

Since the containers are configured to accommodate syringes such as to be separated from one another, it is clear that by using the method and apparatus of the present invention, the syringes that have exited from the packaging machine do not receive any reciprocal impacts up to packing and despatch. In this way, the risks and costs that beset the prior art are completely prevented.

Specific embodiments of the invention, and advantageous technical-functional characteristics related to the embodiments that are only partly derivable from the above description, will be described in the following, in accordance which what is set out in the accompanying claims and with the aid of the appended tables of the drawings, in which:
figure 1 A is an axonometric projection of the apparatus of the invention, associated to a bench of a packaging machine, which is not represented apart from for the endless screw located just within the entrance of the machine;
figure 2 is a schematic view in section and from above of a container for syringes;
figure 3 is a schematic view from above of the pick-up and place device and a part of the gripping device;
figure 4 is a vertical and transversal section of the previous figure;
figure 5 is the view of figure 4 in which the gripping device is activated to collect up the syringe from the pick up and place device;
figure 6 is a schematic view from above of the same situation as the previous figure;
figure 7 is a schematic view in vertical section of a filling cycle of the container for syringes; figure 8 is a schematic view from above of another type of container associated with the pick up and place device and the gripping device;
figure 9 is a schematic representation of the filling cycle of a container of the type illustrated in figure 8;
figures 10 and 11 are views in vertical section and in horizontal section of the act of inserting the syringe into a container of the type shown in figure 8.

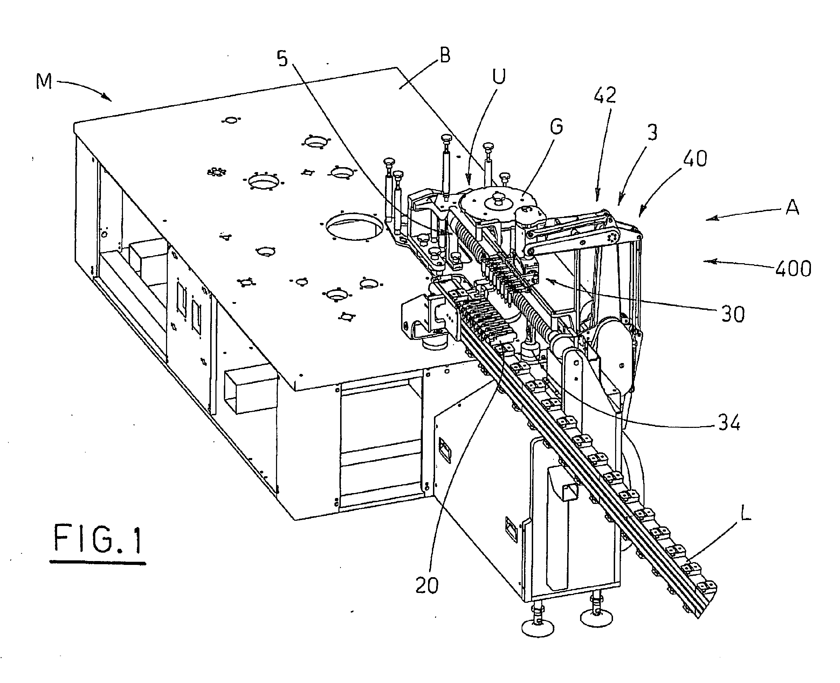

Figure 1 illustrates the apparatus A of the invention, associated to the packaging machine M, with the outlet U thereof exhibiting, immediately internally of the machine M, a carousel which, in known ways, is designed to present each packaged syringe 1 in output in order for it to be collected by the apparatus A of the invention.

Clearly, the representation of the carousel G in the drawings must be understood as auxiliary to the invention: the machine M could be of any type suitable for packaging syringes (and in general articles that are at least partly made of fragile material).

Note that, apart from the carousel G, and the bench B pallet, the machine M is not shown in the appended figures.

The apparatus A, in its possible embodiments, will be described in detail after explaining some possible variants of the method according to the invention.

Primarily, it should be clarified that the syringes 1 may be present in output of the machine M in two configurations.

In a first configuration the syringes 1 comprise a respective body or lateral wall 100 for the liquid to be injected, the edge of the mouth of which forms a collar that, as shall be described, serves as a support element 10 (i.e., expressed in general terms, it is the support element of the article 1 exiting from the machine M).

The lateral wall 100 extends inferiorly into a needle that is completely contained in a safety cap which has a lower end 11 functioning as the bottom of the syringe 1 (i.e. it is the bottom part 11 of the article exiting from the machine M).

This configuration is only schematically illustrated in the enclosed figures, for reasons of simplicity.

In another configuration (not illustrated) the syringe is presented in output from the machine M with the well-known safety device for receiving and constraining the needle once an injection has been performed (commonly called a "safety"), which safety device at least partially comprises the lateral wall 100 of the syringe 1 between its own lasterl walls, also comprises projections on the side thereof (which in general terms identify the support element 10 of the article 1), and finally comprises a lower end which constitutes the bottom 11 of the syringe 1.

In the following, for reasons of simplicity and brevity we will refer simply to syringes 1 having a relative support element 10, a bottom 11 and one (or more) lateral walls 110, in relation both above-cited configurations.

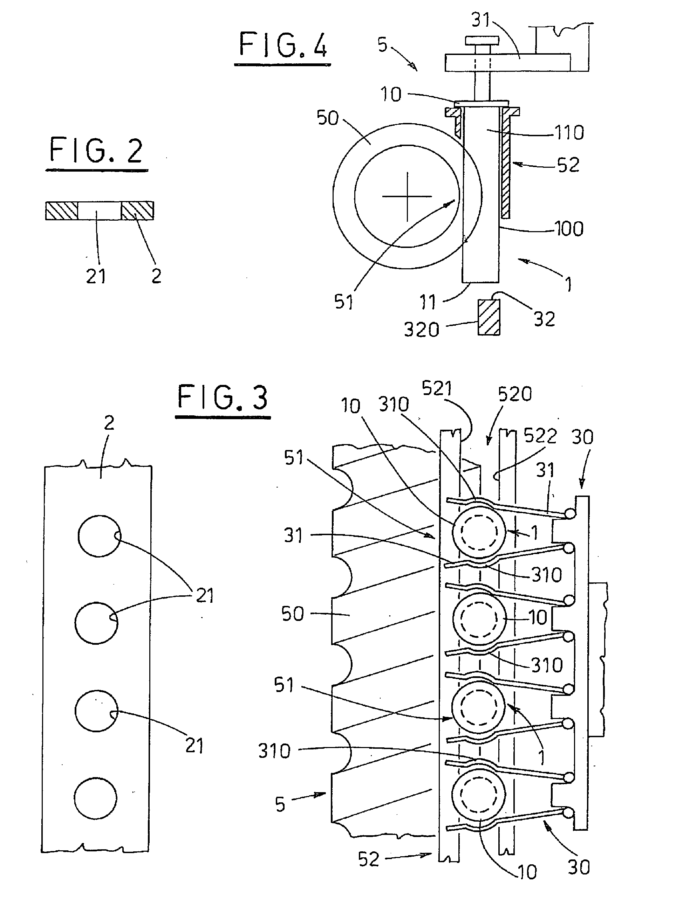

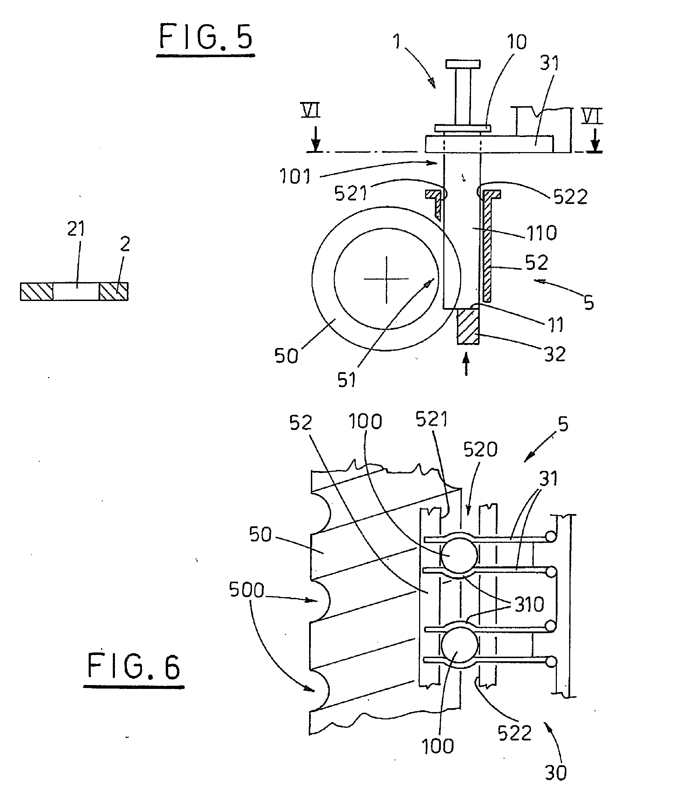

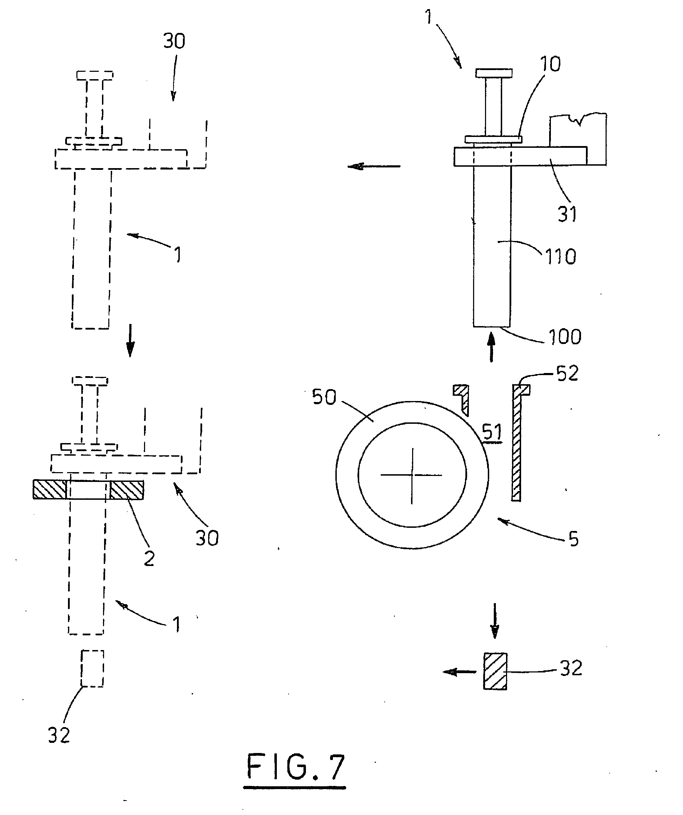

According to the method of claim 1:
during step a., a following sub-step is performed: arranging the articles 1 while they are in the predefined formation, resting on the respective support elements 10 with the bottom 11 thereof left free (this can be understood from schematic figure 4); and
during step b., a following sub-step is performed: pushing the bottom 11 of several articles 1 upwards such as to lift the articles 1 while keeping them resting by means of the bottom 11 thereof, keeping them separate, and such that a respective upper part 101 comprising the support element 10 is lifted and free; and then taking the lifted articles 1 by the upper part 10 thereof, keeping them separated from one another, and taking them to the container 2, 20 (see figures 5 and 7).

In this way, the configurations of the syringes 1 are exploited such as to carry out a transfer that completely prevents any mutual impact.

The containers 2, 20 for syringes 1 are essentially of two types.

The most used is the one that comprises a tray 20 for syringes comprising a plurality of housings 210, in which the syringe 1 can be laid horizontally supported, for example, which housings 210 might be made in a complementary shape to the syringe 1, accessible from above.

The other type of container 2 comprises a series of housings each affording a cylindrical vertical through-cavity where a syringe is housed vertically, resting on the edge of the cavity of the housing through the support element.

The housings 21, 210 are frequently arranged in parallel rows in the container 2, 20, which are often intercalated.

In the case of use of trays, the method may comprise that:
during step a., a following substep is performed: arranging each article 1 vertically when it is in the above-mentioned predefined formation; and in that
before or together with step c., a following step is performed: rotating each article 1 taken in step b. such as to arrange the article 1 horizontally.

This enables laying the syringes 1 horizontally in the respective housing 21, even where the machine M 1 presents syringes 1 vertically in output, as in the case where it comprises the carousel G as described above.

Often a conveyor line L is provided for transporting containers 2, 20. The conveyor line L can be configured such as to move containers 2, 20 in single file, stepped close to the machine M, with the containers 2, 20, one at a time, stopping in the loading position P, up until all the syringes 1 to be housed have been inserted.

Then, the container 2, 20 comprising the syringe 1 is displaced from the loading position P and the conveyor line L is activated to bring another container 2, 20 into the loading position P.

Alternatively, the conveyor line L continuously transports the containers 2, 20.

All the embodiments of the method and apparatus A can be used for both cases, except where explicitly stated otherwise.

Bearing in mind that the housings 21, 210 are arranged in at least a row 2, 20 in the containers, the method may comprise that:
during step a., a following substep is performed: arranging, in the predefined formation, a number of articles/syringes 1 which is at least equal to the number of housings 21, 210 of the line in a container 2, 20,
during step b., a following substep is performed: picking up and taking to the container 2, 20 a number of articles/syringes 1 equal to the number of housings 21, 210 of the line of containers 2, 20,
during step c., a following substep is performed: inserting each syringe 1 picked up and taken to the container in step b. into a respective housing of the line in the container 2, 20;
steps a., b. and c. are repeated applying them cyclically to each of the housings 2, 210 and are actuated such that stage a. has a duration which is at most equal to a sum of a duration of step b. and step c., so that step a. of a cycle in which the articles destined to be inserted in a given line of housings 21, 210 of a container 2, 20 can be completed during steps b. and c. applied in the preceding cycle to a further line of housings 21, 210 of the same container 2, 20 or in a further container; and in that
after respective articles 1 are housed in all the housings 21, 210 of the container 2, 20, the container 2 is distanced by means of the conveyor line L;
whereby the housings 21, 210 of other containers 2, 20 can receive respective articles 1 and the steps of the method can be repeated from the start, for as long as articles 2 are presented at the outlet U of the machine M.

In order to maximize performance, the method provides that, during step a, the following sub-steps are implemented:
distancing the articles/syringes 1 progressively presented at the outlet U of the machine M from the outlet U, such as to form at least a line, comprising a plurality of articles/syringes 1;
moving the line of articles/syringes 1 that have exited the machine M distancing from the outlet U of the machine M, the line being the predefined formation;
moving the line of articles/syringes 1 that have exited the machine M continuously.

The method provides that the following additional step, during step b, is implemented: removing the syringes 1 from the line in a following-mode, while the line is actually moving.

If a conveyor line L is present which transports the containers 2, 20 continuously, step c. can be actuated in the following manner: inserting the articles 1 in the respective housings 21, 210 of a line in a container 2, 20, maintaining them separated from one another and in a following-mode, while the containers are moving borne on the conveyor line L.

This aspect of the method can be combined with the version previously described in which during step b. the syringes 1 are taken from the predefined formation with a following-mode.

In the following possible embodiments of the apparatus A will be described, with functioning modes thereof.

As mentioned, in the apparatus A according to the invention the above-mentioned drawing device 5 comprises a plurality of mobile seatings for singly housing the respective syringes 1 exiting the machine M, and further the pick-up and place device 3 comprises at least a gripping device 30 configured such as to pick up or alternatively release a plurality of syringes 1 and to maintain the gripped syringes 1 separate from one another (see figure 1).

According to a preferred aspect of the invention, the drawing device 3 comprises at least one rest guide 52 (shown in figures 3, 4, 5, 6, 8 and 9) for supporting the syringes 1 presented at the exit from the machine M.

In detail, the rest guide 52 is configured such as to slidingly restingly receive support elements 10 of the syringes 1, leaving the bottom 11 thereof free, and to enable removal thereof from the outlet U of the machine M.

In this way, the apparatus A takes delivery of the syringes 1, advantageously exploiting both the configuration of the syringes and the manner in which they are presented in outlet of the packaging machine M.

Further, in the preferred embodiment, the drawing device 5 comprises at least a screw 50 rotating around a central axis of rotation passing through the output U of the machine M, a first end 50 of the screw being placed at the output U of the machine M.

The screw 50 has, at a lateral periphery thereof, a helical groove 500 (see figures 1, 6 and 8) arranged around the central axis, which groove 500 is dimensioned such as to be able to receive a syringe 1 arranged vertically, abutting the lateral wall 110, of the syringe, the groove 500 further originating from the first end of the Archimedes screw 50 such as to be able to directly accommodate the syringes 1 presented at the outlet U of the machine M.

This aspect of the invention enables translating the syringes 1 away from the outlet U of machine M, in single file and absolutely without any possibility that they might collide against one another.

Taking the above particulars into account, the preferred embodiment of the cited mobile seatings 51 is explained in the following.

Primarily, in this embodiment, the rest guide 52 is straight and is positioned parallel to the rotation axis of the screw 50 (see for example figure 3).

In detail, the guide 52 is arrangeable above the screw 50 in such a way that the groove 500 of the screw 50 defines, together with the rest guide 52, the aforementioned plurality of 51 mobile seatings, which are arranged in a single file along the longitudinal axis of development of the screw 50; in each of the mobile seatings 51 the syringe 1 is housable in the groove 500 and resting on the straight rest guide 52 by means of the respective support element 10 with the respective fund 11 which is left free.

In this embodiment, each mobile seating 51 moves along the longitudinal development of the screw 50, distancing in output from the machine, when the screw 50 is driven in rotation.

The solution described enables immediate delivery of the syringes 1 in outlet from the machine, and supporting them in order to guide them away from the outlet in order that a plurality can be arranged in a predefined formation (in this case, in single file), which is the best for preparing the subsequent displacement thereof and insertion into the containers 2, 20 (as will be explained in the following), all the while ensuring that the syringes 1 can never collide with one another.

According to the preferred constructional choice of the rest guide 52 (for example, illustrated in figure 3 and figure 6), a straight passage 520 is afforded therein, parallel to the rotation axis of the screw 50, defined by two reciprocally-facing lateral edges 521, 522.

The straight passage 520 is arranged above the screw 50 and has a width such that the syringe 1 can be inserted freely between the lateral edges 521, 522 thereof only as far as relative support elements 10 (which again are advantageously exploited to the maximum), so that two opposing sides of each support element 10 are restingly received on a different lateral edge 521, 522, next to the passage 50 of the guide and rest element 52.

This constructional choice is the most economical, functional and least cumbersome possibility.

Before entering into detail with respect to the functioning of the apparatus A, it is well to specify what is the preferred embodiment of the above-mentioned drawing means 30.

As can be seen in almost all the figures from 3 to 10, the invention comprises a plurality of pliers 30, each of which is closable for solidly gripping an article 1 or alternatively openable for releasing the article, the pliers 30 being arranged at a reciprocal distance such that the articles 1, while being picked up and once picked up by a respective pliers 30, are maintained separated from one another.

Thanks to this detail, during the critical passage from the drawing device 5 to the container 2, 20 no mutual collision is possible between the syringes 1, and this while maintaining the operational constraint to transfer a variety of syringes 1 at a time. Preferably, each pliers 30 comprises a pair of jaws 31 that can be reciprocally rotated with respect to an axis of rotation, alternatively for approaching, closing the pliers 30, or distancing, opening the pliers 30.

The jaws 31 are shaped such that when the pliers 30 close on a syringe 1 they can couple thereto, solidly tightening the lateral wall 110 thereof between the jaws 31.

To understand the functioning of the apparatus A it is necessary to introduce another particular of the invention, which is designed such as to collaborate functionally with the screw 50, with the above-described guide above 52 and with the pliers 30.

The pick-up means 3 can comprise a thrust element 32 (schematically illustrated in figures 4, 5 and 7, for example), arranged inferiorly of the drawing device 5.

The thrust element 32, which comprises a top abutting surface 320, can oscillate vertically in such a way that, when performing an upwards run, it can push, with the upper surface 320 thereof, the free bottom 11 of the syringes 1 housed in the mobile seatings 52, causing a lifting of the aforementioned upper part 101 (see figure 5) which, as has been mentioned, comprises the support element 10.

With the upper part 101 of the syringes 1 raised, it projects superiorly of the mobile seating 51.

In particular, the pusher element can comprise a rod 32 or a plate, having the upper abutting surface horizontal and mounted such as to lie in a common vertical plane with the plurality of pliers 30 flanked.

The functioning of A is described below.

The drawing device 5, preferably comprising the screw 50 and guide 52, draws away the syringes 1 in output from the machine M.

The syringes are preferably 1 arranged in a single file that moves, for example, continuously. The pusher element 32 locates below the syringes 1 still housed in the mobile seatings 51, while the plurality of pliers 30 locates above (see figure 4), in a manner which will be more fully explained herein below.

The pusher element 32 upwardly pushes a certain number of syringes 1 (figure 5), preferably equal in number to the housings 21, 210 of a row of containers 2, 20. As shown in figure 5, immediately following the push, or simultaneously with it, the pliers 30 each take a syringe 1 (preferably the number of pliers 30 will be equal to the number of the housings 21, 210 in a row of containers 2, 20).

After they have been coupled tightly to the syringe 1, the pliers 30 are moved such as to take them away from the mobile housings 51 (right side of figure 7, figure 9 and figure 10), and then to bring them to the containers 2, 20 where they are inserted in the respective housing 21, 210 (as seen on the left side of figures 7, 9 and 10).

The pick-up device 3 then returns the pliers 30 to the drawing device 5.

In practice, the apparatus operates a succession of cycles of drawing and filling in each of which it takes an entire row of syringes 1 from the machine M, and then inserts the syringes 1 into the container 2, 20, up until, after a certain number of cycles, all the housings 21, 210 of the container 2, 20 are filled and the apparatus can return to fill a row of housings 21, 210 of the container 2, 21 in the next cycle.

As with the apparatus A in its fundamental parts as described above, it has a functioning which is such as to completely ensure that during transfer from the outlet U of the machine M to the container 21, 210, no impacts will occur between the syringes 1.

To further clarify the critical phase of the extraction of the syringes 1 from the drawing device 5, it is specified that the plurality of pliers 30 is arrangeable on the drawing device 5 in such an arrangement that they are activatable to grip the respective syringe 1 pushed upwards by the thrust element 32, tightly retaining said syringe 1 by the upper projecting part 101 thereof.

The plurality of pliers 30 is preferably arranged in a straight horizontal line, with the axes of rotation 31 of the jaws parallel to each other.

In a second preferred aspect illustrated in figures 3, 6 and 8, for example, each jaw 31 of each pair has a gripping section 310, facing the gripping section 310 of the other jaw 31 of the pair, and having a shape that is complementary to that of the lateral wall 100 of the syringe 1.

Even more specifically, the jaws 31, when tightening on the respective syringe 1, can be located horizontally with respect thereto such as to grip the upper projecting part 101 of the respective syringe 1, as already mentioned, and at the same time, such as to inferiorly abut the support element 10 itself, in such a way as to increase the firmness of the grip (this detail is not shown, but its can be derived from what has been described herein above).

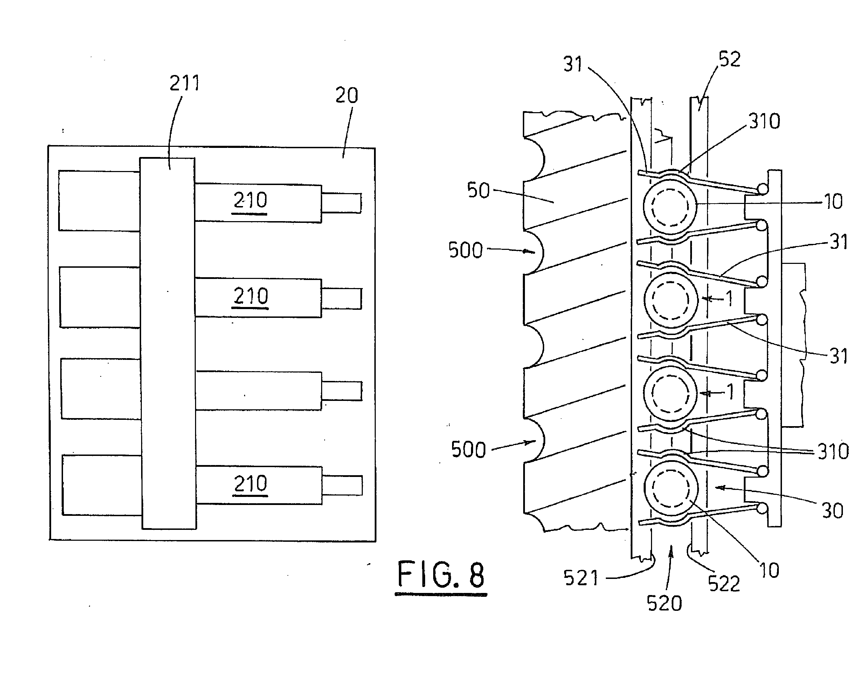

As explained above, the containers for syringes 1 may comprise trays 20 as described above in detail (and illustrated in figure 8).

In this case, the invention can comprise an embodiment of the apparatus (shown schematically in figures 8, 9 and 10), in which each pliers 30 comprises a pair of jaws 31 of such dimensions as to fit into a housing 210 in order to grip a syringe 1.

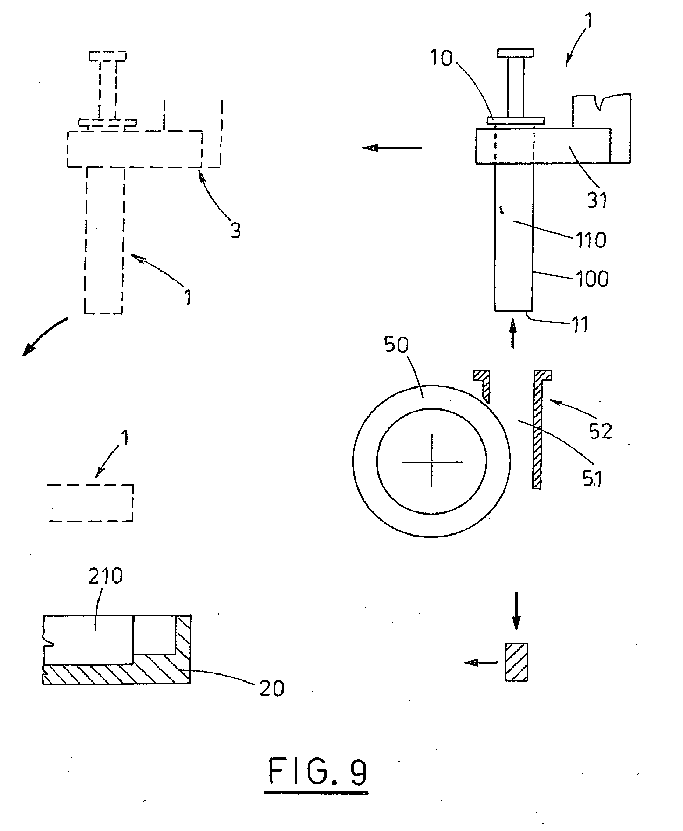

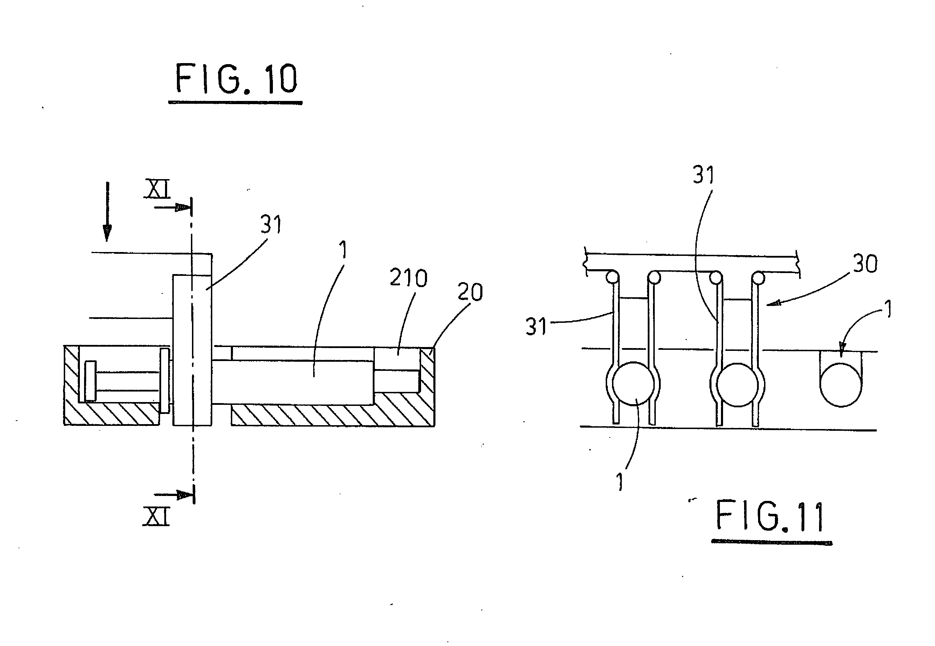

In this case, pick-up means 3 further comprises a rotation device connected to the plurality of pliers 30 in order to rotate the syringes 1 (see figure 9) picked up by the pick-up device 3 and arrange them horizontally and insert them in the relative housings 210 of the tray (see figure 10).

For example, the rotating device can be formed by a motor connected to the plurality of pliers 30 and inserted into an operating head 33, which supports the whole row of pliers 30, or by other equivalent devices known to the person skilled in mechanical engineering and the like.

Not infrequently, and this is well-known, the housings 201 of the trays extend laterally (see figure 8) such as to create, by the side of each housed syringe 1, an undercut 211, i.e. an empty space for facilitating picking-up and collecting of the syringes 1.

In this case, each jaw 31 of a pliers 30 can be of such dimensions as to fit into the undercut 211 so that the pliers 30 can take a respective syringe 1 by tightening on the lateral wall 110 thereof.

In an aspect illustrated only in figure 1, the pick-up and place device 3 preferably comprises a robot 400 of known type for supporting and moving the plurality of pliers 30.

This type of robot 400 is of known type and serves to support and move an object in different points of space.

Several types of robots of this type are known *per* se, but none in combination with other aspects of the apparatus A.

In a particular embodiment of the robot 400, said robot 400 comprises:
a bearing body 40 destined to oscillate between loading positions thereof, by a flank of the pick-up device, and an unloading position thereof, in proximity of a container transported by the conveyor line,
a guide 41 for constraining oscillation of the bearing body 40,
a motor for displacing the bearing body 40 along the guide 41 or for halting the bearing body 40 at any position along the guide 41; and
a support arm 42 solidly mounted to the bearing body 40, on which support arm 42 the plurality of pliers 30 is mounted, the support arm 42 being movable such as to displace the pliers 30 along two Cartesian axes of a vertical plane.

The support arms 42 of the type suitable for moving the pliers 30 in the manner specified are known in themselves but not in combination with other aspects of the pick-up device 3 and even less known with the mentioned additional aspects and to be described of the apparatus A.

The supporting body 40, when in the loading position, is arranged above the drawing device 5 so that the support arm 42 can raise the pliers 30, once they have gripped the syringes 1, such as to extract them from the mobile housings 51.

Further, the supporting body 40, when in the unloading position, is arranged, with respect to the conveyor line L, in such a way that the arm 42 can lower the pliers 30 bearing the syringes 1 so that the syringes 1 can be inserted into respective housings 21, 210 in a given container 2,10 to which they are destined.

After the syringes 1 have been removed from the mobile housings, thanks to the movement of the supporting body 40 along the guide, they are brought to the container 2, 20 where they are inserted into the housings 21, 210.

At this point, the pick up and place cycle recommences by being applied to a further row of syringes 1 exiting the machine M.

In practice, after the insertion of needles into the housings 21, 210 also referred to herein as "filling", the robot 4 brings the supporting body 40 back to the drawing device 5.

When after a given number of repeated cycles, which in practice is equal to the parallel rows of housings 21, 210 in the container 2, 20, the container 2, 20 is completely filled, then a further container 2, 20 to be filled is started on.

Finally, the pick-up and place device 5 comprises a pusher organ 34 mounted solidly to the supporting body 40 of the robot 400 and fixed inferiorly to the thrust element 32, the pusher organ (34) being operable to cause the vertical oscillation of the thrust element 32, which is mounted above such that the upper surface 320 thereof is vertically below the line of pliers 30.

The pusher organ (34) can be a jack, a linear actuator or other known equivalent device. Finally, in a possible constructional aspect, the guide of the robot 400 comprises a straight beam for supporting the supporting body 40 such that it only runs along the longitudinal development of the beam.

The foregoing has been described by way of non-limiting example, and any constructional variants are understood to fall within the ambit of the present technical solution, as claimed in the following.

## Claims

1. A method for transferring a plurality of articles (1), at least partially made of a fragile material, for example syringes for injections, from an automatic machine (M) which presents them to an outlet (U) thereof separated from one another, to containers (2, 20) comprising a plurality of housings (21, 210) in each of which the articles are singly housable separately from one another, wherein each of the articles (1) comprises a support element (10) which projects laterally, and a bottom (11) at a lower end thereof, the method comprising the following steps:
a. arranging a plurality of articles (1) presented at the outlet (U) of the automatic machine (M) in a predefined formation in which they are maintained separated from one another;
b. removing more than one of the articles (1) arranged in step a. and maintaining them separated from one another, taking them to a container (2, 20); and
c. inserting the articles (1), maintaining them separated from one another, into the container (2, 20), and wherein:
- during step a., a following sub-step is performed: arranging the articles (1) while they are in the predefined formation, resting on the respective support elements (10) with the bottom (11) thereof left free, and the following substeps are performed:
distancing the articles (1) progressively presented at the outlet (U) of the machine (M) from the outlet (U), such as to form at least a line, comprising a plurality of articles (1);
moving the line of articles (1) that have exited the machine (M) distancingly from the outlet (U) of the machine (M), the line being the predefined formation;
moving the line of articles (1) that have exited the machine (M) continuously;
- during step b., a following sub-step is performed: pushing the bottom (11) of several articles (1) upwards such as to lift the articles (1) while keeping them resting by means of the bottom (11) thereof, keeping them separate, and such that a respective upper part (101) comprising the support element (10) is lifted and free; and then taking the lifted articles (1) by the upper part (101) thereof, keeping them separated from one another, removing the articles (1) from the line in a following-mode, while the line is actually moving and taking them to the container (2, 20).

2. The method of the preceding claim, usable for transferring elongate articles (1) at least partially made of a fragile material to containers each comprising a tray (20) for containing the elongate articles (1), in turn comprising a plurality of open housings (210) for singly accommodating respective articles (1) lying horizontally, the housings (210) being arranged with respect to one another in such a way as to be separated from one another, the method being **characterised in that**:
during step a., a following substep is performed: arranging each article (1) vertically when it is in the above-mentioned predefined formation; and **in that** before or together with step c., a following step is performed: rotating each article (1) taken in step b. such as to arrange the article (1) horizontally.

3. The method of any one of the preceding claims, usable when a conveyor line (L) is provided for transporting the containers (2, 20), presenting containers (2, 20) without articles (1) and distancing them once all the housings (21, 210) thereof house respective articles (1), in which containers (2, 20) the housings (21, 210) are arranged in at least a line, the method being **characterised in that**:
during step a., a following substep is performed: arranging, in the predefined formation, a number of articles (1) which is at least equal to the number of housings (21, 210) of the line in a container (2, 20),
during step b., a following substep is performed: picking up and taking the container (2, 20) a number of articles (1) equal to the number of housings (21, 210) of the line of containers (2, 20),
during step c., a following substep is performed: inserting each articles picked up and taken to the container in step b. into a respective housing of the line in the container,
steps a., b. and c. are repeated applying them cyclically to each of the housings (2, 210) and are actuated such that stage a. has a duration which is at most equal to a sum of a duration of step b. and step c., so that step a. of a cycle in which the articles destined to be inserted in a given line of housings (21, 210) of a container (2, 20) are predisposed can be completed during steps b. and c. applied in the preceding cycle to a further line of housings (21, 210) of the same container (2, 20) or in a further container; and **in that** after respective articles (1) are housed in all the housings (21, 210) of the container (2, 20), the container (2) is distanced by means of the conveyor line (L);
whereby the housings (21, 210) of other containers (2, 20) can receive respective articles (1) and the steps of the method can be repeated from the start, for as long as articles (2) are presented at the outlet of the machine (M).

4. The method of claim 3, usable when the conveyor line (L) transports the containers (2, 20) continuously, wherein step c. is actuated as follows:
inserting the articles (1) in the respective housings (21, 210) of a line in a container (2, 20), maintaining them separated from one another and in a following-mode, while the containers are moving borne on the conveyor line (L).

5. An apparatus for transferring a plurality of elongate articles (1) at least partially made of a fragile material, such as for example syringes for injections, each comprising one or more lateral walls (110), a support element (10) which projects laterally, and a bottom (11) at a lower end thereof, by an automatic machine (M), which presents the articles (1) at an outlet (U) thereof separated from one another, to containers (2, 20) comprising a plurality of housings (21, 210) in each of which the articles (1) can be singly accommodated so as to be separated from one another, with the containers (2, 20) which are transported by a conveyor line (L) which presents the containers (2, 20) without articles (1) and distances the containers once all the housings (21, 210) thereof accommodate respective articles (1), in which containers the housings are arranged in at least a line, the apparatus comprising:
at least a drawing device (5) for receiving and taking delivery of a plurality of articles (1) which exit the machine (M) as and when they are presented at the outlet (U) thereof, the drawing device (5) comprises at least a rest guide (52) for supporting the articles (1) presented at the outlet (U) of the machine (M), the rest guide (52) being configured such as to be able to restingly slidingly receive the support elements (10) of the articles, leave a bottom (11) thereof free and enable distancing thereof from the outlet (U) of the machine (M) and a plurality of mobile seatings (51) for singly housing respective articles (1) presented at the outlet (U) of the machine (M) and moves the housed articles (1) distancingly from the outlet (U) of the machine (M), the mobile seatings (51) being arranged with respect to one another in such a way that the articles (1) housed therein are maintained separated from one another,
at least a pick-up and place device (3) for removing the articles from the drawing device (5), taking them to the container (2, 20) and inserting them in respective housings (21, 210) thereof, the pick-up and place device (3) comprising at least a gripping means (30) configured such as to be able to pick up or alternatively release a plurality of articles (1) and to maintain the picked-up articles separate from one another, the gripping means comprising a plurality of pliers (30), each of which is closable for solidly gripping an article (1) or alternatively openable for releasing the article, the pliers (30) being arranged at a reciprocal distance such that the articles (1), while being picked up and once picked up by a respective pliers (30), are maintained separated from one another,
the apparatus (A) being **characterised in that**:
the pick-up means (3) comprises at least a thrust element (32), arrangeable inferiorly of the pick-up device and comprising an upper abutting surface (320), the thrust element (32) being able to oscillate vertically such that when it performs an upwards run it can push, with the upper surface (320) thereof, the free bottom (11) of articles housed in the mobile seatings, causing an upper part (101) of the pushed articles (1) comprising the support element (10) to project superiorly of the mobile seating and the rest guide (21), the plurality of pliers (30) is arrangeable above the pick-up device; and **in that** each of the relative pliers (30) is closable in order to grip one of the articles (1) pushed upwards by the thrust element (32), solidly gripping it by the upper projecting part (101) thereof, or is alternatively openable in order to release the article (1),
said pick-up and place device (3) comprises a robot (4) for supporting and moving the plurality of pliers (30) in space, with the robot (400) which comprises:
a bearing body (40) destined to oscillate between loading positions thereof, by a flank of the pick-up device, and an unloading position thereof, in proximity of a container transported by the conveyor line,
a guide (41) for constraining oscillation of the bearing body (40),
a motor for displacing the bearing body (40) along the guide (41) or for halting the bearing body (40) at any position along the guide (41); and
a support arm (42) solidly mounted to the bearing body (40), on which support arm (42) the plurality of pliers (30) is mounted, the support arm (42) being movable such as to displace the pliers (30) along two Cartesian axes of a vertical plane;
when in the loading position, the bearing body (40) being arranged with respect to the drawing device (5) such that the support arm (42) can lift the pliers (30), once the pliers (30) have gripped the articles (1), such as to extract the articles (1) from the respective mobile seatings (51), and the bearing body (40), when in the unloading position, being further arranged with respect to the conveyor line (L) such that the arm (42) can lower the pliers (30) which bear the articles in order for the articles (1) to be inserted in respective housings of a container to which they are destined, and **in that** the pliers (30) are arranged in a horizontal line; the pick-up and place device (3) further comprises a pusher organ (34) solidly mounted to the bearing body (40) of the robot (400) and inferiorly fixed to the thrust element (32), activatable to cause the vertical oscillation of the thrust organ (32); and **in that** the thrust element (32) is mounted on the pusher organ (34) such that the upper abutting surface (320) is vertically below the line of pliers (30).

6. The apparatus of the preceding claim, wherein the drawing device (5) comprises at least an Archimedes screw (50) rotatable about a central rotating axis which passes through the outlet (U) of the machine (M), with a first end of the Archimedes screw (50) being locatable at the outlet (U) of the machine (M), the Archimedes screw having, at a lateral periphery thereof, a helical groove (500) arranged about the central axis, which groove (500) is dimensioned such as to be able to receive an article (1) arranged vertically, abutting the lateral wall (110) of the article (1), the groove (500) further originating from the first end of the Archimedes screw (50) such as to be able to directly accommodate the articles (1) presented at the outlet (U) of the machine (M).

7. The apparatus of the preceding claim, **characterised in that** the rest guide (52) is straight, is positionable parallel to the rotation axis of the Archimedes screw (50) and is further arrangeable with respect thereto such that the groove (500) of the Archimedes screw (50) defines, together with the rest guide (52), the plurality of mobile seatings (51), which are arranged in a line along the longitudinal development of the Archimedes screw (50), each article (1) being housable in each of the mobile seatings (51) accommodated in the groove (50) and resting on the straight guide (52) on the respective support element (10) with the free bottom (11), each mobile seating (51) moving along the longitudinal development of the Archimedes screw (50), distancingly from the outlet of the machine (M) when the Archimedes screw (50) is activated in rotation.

8. The apparatus of the preceding claim, wherein a straight passage (520) is afforded in the rest guide (52), which straight passage (520) is parallel to the axis of symmetry of the Archimedes screw (50) and is defined by two lateral edges (521, 522) facing one another, the straight passage (530) being arranged above the Archimedes screw (50) and having a width which is such that the articles (1) can be freely inserted between the lateral edges (521, 522) only up to relative support elements (10) thereof, such that two opposite sides of each support element (10) are each received restingly on a different lateral edge (521, 522) flanking and defining the passage (50) of the guide and rest element (52).

9. The apparatus of the preceding claim, usable for transferring elongate articles (1) comprising one or more lateral walls (110), the apparatus (A) being **characterised in that** each pliers (30) comprises a pair of jaws (31) that are reciprocally rotatable with respect to a rotation axis, alternatively to near and thus close the pliers (30) or to distance and open the pliers (30), the jaws (31) having a shape such that, when the pliers (30) close, an article (1) can be coupled thereto, solidly gripping the lateral wall (100) which is gripped between the jaws (31).

10. The apparatus of claim 5, for transferring articles (1) to containers each comprising a tray (20) for articles (1) in a fragile material, in turn comprising a plurality of open housings (210), accessible superiorly, and able to singly house the articles (1) lying horizontally so that the articles (1) are separated from one another, the apparatus (A) being **characterised in that** each pliers (30) comprises a pair of jaws (31) that are dimensioned such as to insert in a housing (210) in order to grip an article (1); and further the gripping means comprises a rotation device connected to the plurality of pliers (30) such as to be able to rotate the articles (1) picked up from the drawing device (5) such as to arrange them horizontally in order to be able to insert them in the relative housings (210) of the tray (20).

11. The apparatus of one of the claims 5, 6 or 9, usable for transferring articles (1) which each comprise a syringe for injections, which syringe comprises a barrel (100) for the liquid to inject, the upper element (10) is a collar formed at a rim of a mouth of the tube (100), and the bottom (11) of which is a lower end of a safety cap which houses a needle of the syringe, the apparatus (A) being **characterised in that** the rest guide (52), the groove (500) of the Archimedes screw (50), the jaws (31) of the pliers (30) and the thrust element (32) are dimensioned such as to able respectively to: restingly slidingly receive the collars of the barrels of the syringe, accommodate the syringes by abutting a lateral wall thereof, solidly grip the lateral wall of the syringes and push the lower end of the safety cap.

12. The apparatus of one of the claims 5, 6 or 9, usable for transferring articles (1) which each comprise a syringe for injections and a safety device for housing and retaining the needle once an injection has been made, which safety device at least partially includes the barrel (100) of the syringe between lateral walls thereof, comprises lateral projections which constitute the support element (10) of the article and finally comprises a lower end which constitutes the bottom (11), the apparatus (A) being **characterised in that** the rest guide (52), the groove (500) of the Archimedes screw (50), the jaws (31) of the pliers (30) and the thrust element (32) are dimensioned such as respectively to be able to: restingly sliding receive the lateral projections of the safety device, accommodate the safety device by abutting a lateral wall thereof, solidly grip the lateral wall of the safety device and push the lower end of the safety device.

## Patentansprüche

1. Verfahren zum Überführen von mehreren Artikeln (1), die zumindest teilweise aus einem zerbrechlichen Material bestehen, wie zum Beispiel Spritzen für Injektionen, von einer automatischen Maschine (M), welche die Artikel an einem Auslauf (U) der Maschine voneinander beabstandet darbietet, zu Behältern (2, 20), die mehrere Aufnahmefächer (21, 210) beinhalten, in denen die Artikel jeweils einzeln und getrennt voneinander untergebracht werden können, worin jeder der Artikel (1) ein seitlich hervorstehendes Halteelement (10) und eine Unterseite (11) an seinem unteren Ende beinhaltet; wobei das Verfahren **dadurch gekennzeichnet ist, dass** es folgende Schritte beinhaltet:
a. Anordnen mehrerer Artikel (1), die am Auslauf (U) der automatischen Maschine (M) dargeboten werden, in einer vorbestimmten Formation, in der sie voneinander beabstandet gehalten werden;
b. Entnehmen von mehr als einem der in Schritt a. angeordneten Artikel (1) und Überführen dieser weiterhin voneinander beabstandet gehaltenen Artikel zu einem Behälter (2, 20); und
c. Einlegen der weiterhin voneinander beabstandet gehaltenen Artikel (1) in den Behälter (2, 20), wobei:
- während Schritt a. ein folgender Unterschritt ausgeführt wird: Anordnen der Artikel (1), während sie sich in der vorbestimmten Formation befinden, so dass sie auf den entsprechenden Halteelementen (10) aufliegen und ihre Unterseite (11) jeweils frei bleibt, und die folgenden Unterschritte ausgeführt werden:
Entfernen der nach und nach am Auslauf (U) der Maschine (M) dargebotenen Artikel (1) von dem Auslauf (U), um zumindest eine Reihe zu bilden, in der mehrere Artikel (1) enthalten sind;
Befördern der Reihe von aus der Maschine (M) ausgelaufenen Artikeln (1) weg von dem Auslauf (U) der Maschine (M), wobei diese Reihe die genannte vorbestimmte Formation darstellt;
Befördern der Reihe von aus der Maschine (M) ausgelaufenen Artikeln (1) mit kontinuierlicher Bewegung;
- während Schritt b. ein folgender Unterschritt ausgeführt wird:
Schieben der Unterseite (11) mehrerer Artikel (1) nach oben, um die Artikel (1) anzuheben, wobei sie weiterhin auf ihrer Unterseite (11) aufliegen bleiben und voneinander beabstandet gehalten werden, so dass ein jeweiliger oberer Teil (101), der das Halteelement (10) beinhaltet, angehoben und frei zugänglich wird; und anschließendes Ergreifen der angehobenen Artikel (1) an ihrem oberen Teil (101), um sie weiterhin voneinander beabstandet zu halten, sowie Entfernen der Artikel (1) aus der Linie im Verfolgermodus, während der Weiterbewegung der Linie und Überführen der Artikel zu dem Behälter (2, 20).

2. Verfahren nach dem vorhergehenden Anspruch, anwendbar zum Überführen von länglichen Artikeln (1), die zumindest teilweise aus einem zerbrechlichen Material bestehen, zu Behältern, die jeweils ein Tray (20) zur Aufnahme der länglichen Artikel (1) beinhalten, welches seinerseits mehrere offene Aufnahmefächer (210) zum einzelnen Aufnehmen entsprechender horizontal darin eingelegter Artikel (1) beinhaltet, wobei diese Aufnahmefächer (210) derart zueinander angeordnet sind, dass sie durch einen Abstand voneinander getrennt sind, wobei das Verfahren **dadurch gekennzeichnet ist, dass**:
während Schritt a. ein folgender Unterschritt ausgeführt wird: vertikales Anordnen jedes Artikels (1), wenn er sich in der genannten vorbestimmten Formation befindet; und dadurch, dass
vor oder zusammen mit Schritt c. ein folgender Schritt ausgeführt wird:
Drehen jedes in Schritt b. aufgenommenen Artikels (1), um den Artikel (1) horizontal anzuordnen..

3. Verfahren nach einem der vorhergehenden Ansprüche, anwendbar, wenn eine Fördererlinie (L) zum Befördern der Behälter (2, 20) vorgesehen ist, welche die Behälter (2, 20) ohne Artikel (1) darbietet und diese dann abtransportiert, nachdem in allen entsprechenden Aufnahmefächern (21, 210) jeweils Artikel (1) untergebracht sind, wobei in den Behältern (2, 20) die Aufnahmefächer (21, 210) in zumindest einer Reihe angeordnet sind, wobei das Verfahren **dadurch gekennzeichnet ist, dass**:
während Schritt a. ein folgender Unterschritt ausgeführt wird: Anordnen einer Anzahl von Artikeln (1), die zumindest gleich der Anzahl von Aufnahmefächern (21, 210) der Reihe in einem Behälter (2, 20) ist, in der vorbestimmten Formation,
während Schritt b. ein folgender Unterschritt ausgeführt wird: Aufnehmen einer Anzahl von Artikeln (1), die zumindest gleich der Anzahl von Aufnahmefächern (21, 210) der Reihe in einem Behälter (2, 20) ist, und deren Überführen zu dem Behälter (2, 20),
während Schritt c. ein folgender Unterschritt ausgeführt wird: Einlegen der in Schritt b. aufgenommenen und zu dem Behälter überführten Artikel in ein entsprechendes Aufnahmefäch der Reihe in dem Behälter,
die Schritte a., b. und c. wiederholt werden, indem sie zyklisch auf alle Aufnahmefächer (21, 210) angewendet und derart ausgeführt werden, dass der Schritt a. eine Dauer aufweist, die so weit wie möglich gleich der Summe der Dauer von Schritt b. und Schritt c. ist, so dass Schritt a. eines Zyklus zum Anordnen der zum Einlegen in eine vorgegebene Reihe von Aufnahmefächern (21, 210) eines Behälters (2, 20) bestimmten Artikel ausgeführt werden kann, während die Schritte b. und c. des vorhergehenden Zyklus auf eine weitere Reihe von Aufnahmefächern (21, 210) desselben Behälters (2, 20) oder eines weiteren Behälters angewendet werden; und dadurch, dass
nachdem entsprechende Artikel (1) in allen Aufnahmefächern (21, 210) des Behälters (2, 20) untergebracht sind, der Behälter (2) mittels der Fördererlinie (L) abtransportiert wird;
wobei die Aufnahmefächer (21, 210) anderer Behälter (2, 20) entsprechende Artikel (1) aufnehmen können und die Schritte des Verfahrens von Anfang an wiederholt werden können, so lange Artikel (1) am Auslauf der Maschine (M) dargeboten werden.

4. Verfahren nach Anspruch 3, anwendbar, wenn die Fördererlinie (L) die Behälter (2, 20) kontinuierlich befördert, worin Schritt c. wie folgt ausgeführt wird: Einlegen der weiterhin voneinander beabstandet gehaltenen Artikel (1) in die entsprechenden Aufnahmefächer (21, 210) einer Reihe in einem Behälter (2, 20) im Verfolgermodus, während die von der Fördererlinie (L) getragenen Behälter weiterbefördert werden.

5. Vorrichtung zum Überführen von mehreren länglichen Artikeln (1), die zumindest teilweise aus einem zerbrechlichen Material bestehen, wie zum Beispiel Spritzen für Injektionen, die jeweils eine oder mehrere Seitenwände (110), ein seitlich hervorstehendes Halteelement (10) und eine Unterseite (11) an ihrem unteren Ende aufweisen, durch eine automatische Maschine (M), welche an ihrem Auslauf (U) die Artikel (1) voneinander beabstandet darbietet, zu Behältern (2, 20), die mehrere Aufnahmefächer (21, 210) beinhalten, in welchen die Artikel (1) jeweils einzeln aufgenommen werden können, um voneinander beabstandet zu bleiben, wobei die Behälter (2, 20) durch eine Fördererlinie (L) befördert werden, welche die Behälter (2, 20) ohne Artikel (1) darbietet und welche die Behälter, sobald in allen Aufnahmefächern (2, 20) entsprechende Artikel (1) untergebracht sind, abtransportiert, und worin die Aufnahmefächer in den Behältern in zumindest einer Reihe angeordnet sind, wobei die Vorrichtung Folgendes beinhaltet:
zumindest eine Entnahmeeinrichtung (5) zum Empfangen und Übernehmen von mehreren aus der Maschine (M) auslaufenden Artikeln (1), während diese nach und nach am Auslauf (U) der Maschine dargeboten werden, wobei die Entnahmeeinrichtung (5) beinhaltet:
zumindest eine Auflageführung (52) zum Tragen der Artikel (1), die am Auslauf (U) der Maschine (M) dargeboten werden, wobei diese Auflageführung (52) derart konfiguriert ist, dass sie die Halteelemente (10) der Artikel gleitbar aufliegend aufnehmen kann, deren Unterseite (11) frei lassen kann und deren Abtransportieren von dem Auslauf (U) der Maschine (M) ermöglichen kann; sowie mehrere bewegliche Aufnahmen (51) zum einzelnen Aufnehmen entsprechender Artikel (1), die am Auslauf (U) der Maschine (M) dargeboten werden, und Bewegen der aufgenommenen Artikel (1) weg vom Auslauf (U) der Maschine (M), wobei diese beweglichen Aufnahmen (51) derart relativ zueinander angeordnet sind, dass die darin aufgenommenen Artikel (1) voneinander beabstandet gehalten werden;
zumindest eine Aufgreif und Ablageeinrichtung (3), die dazu dient, die Artikel aus der Entnahmeeinrichtung (5) zu entnehmen, sie zu dem Behälter (2, 20) zu überführen und in dessen entsprechende Aufnahmefächer (21, 210) einzulegen, wobei die Aufgreif und
Ablageeinrichtung (3) zumindest ein Greifinittel (30) beinhaltet, das derart konfiguriert ist, dass es in der Lage ist, mehrere Artikel (1) abwechselnd zu ergreifen oder freizugeben und die aufgegriffenen Artikel voneinander beabstandet zu halten, wobei die Greifinittel mehrere Greifzangen (30) beinhalten, die jeweils geschlossen werden können, um einen Artikel (1) fest zu greifen, oder abwechselnd dazu geöffnet werden können, um den Artikel freizugeben, wobei die Greifzangen (30) in einem solchen Abstand zueinander angeordnet sind, dass die Artikel (1), während sie aufgenommen werden und von einer Greifzange (30) gegriffen werden, voneinander beabstandet gehalten werden,
wobei die Vorrichtung (A) **dadurch gekennzeichnet ist, dass**:
die Greifinittel (3) zumindest ein Schubelement (32) beinhalten, das unterhalb der Aufgreifeinrichtung angeordnet ist und eine obere Anschlagfläche (320) aufweist, wobei das Schubelement (32) in vertikaler Richtung derart hin und her bewegt werden kann, dass es bei seiner Aufwärtsbewegung mit seiner oberen Anschlagfläche (320) die freie Unterseite (11) der in den beweglichen Aufnahmen befindlichen Artikel so nach oben schieben kann, dass der obere Teil (101) der geschobenen Artikel (1), der das Halteelement (10) beinhaltet, oben von der beweglichen Aufnahme und der Auffageführung (52) hervorragt,
die mehreren Greifzangen (30) oberhalb der Aufgreifeinrichtung angeordnet werden können; und dass
jede der entsprechenden Greifzangen (30) geschlossen werden kann, um einen der von dem Schubelement (32) nach oben geschobenen Artikel (1) aufzunehmen, indem sie ihn an seinem hervorstehenden oberen Teil (101) ergreift, oder wechselweise geöffnet werden kann, um den Artikel (1) freizugeben,
wobei die Aufgreif und Ablageeinrichtung (3) einen Roboter (4) zum Tragen und Bewegen der mehreren Greifzangen (30) im Raum beinhaltet, wobei der Roboter (40) Folgendes beinhaltet:
einen tragenden Körper (40), der dafür ausgelegt ist, sich zwischen einer Ladestellung auf einer Seite der Aufgreifeinrichtung und einer Entladestellung in Nähe eines von der Fördererlinie beförderten Behälters hin und her zu bewegen,
eine Führung (41) zur Festlegung der Hin- und Herbewegung des tragenden Körpers (40),
einen Motor zum Bewegen des tragenden Körpers (40) entlang der Führung (41) oder zum Anhalten des tragenden Körpers (40) an einer beliebigen Position entlang der Führung (41); und
einen Trägerarm (42), der fest am tragenden Körper (40) montiert ist, wobei auf dem Trägerarm (42) mehrere Greifzangen (30) montiert sind und der Trägerarm (42) derart beweglich ist, dass er die Greifzangen (30) entlang zweier kartesischer Achsen einer vertikalen Ebene bewegen kann;
wobei der tragende Körper (40), wenn er sich in der Ladestellung befindet, relativ zu der Entnahmeeinrichtung (5) so angeordnet ist, dass der Trägerarm (42) die Greifzangen (30) anheben kann, sobald die Greifzangen (30) die Artikel (1) ergriffen haben, um die Artikel (1) aus den entsprechenden beweglichen Aufnahmen (51) abzuziehen, und wobei der tragende Körper (40) ferner, wenn er sich in der Entladestellung befindet, relativ zu der Fördererlinie (L) so angeordnet ist, dass der Arm (42) die Greifzangen (30), welche die Artikel tragen, absenken kann, um die Artikel (1) in entsprechende Aufnahmefächer eines Behälters, für den sie bestimmt sind, einzulegen,
und dadurch, dass die Greifzangen (30) in einer horizontalen Linie angeordnet sind;
wobei die Aufgreif und Ablageeinrichtung (3) ferner ein Schieberorgan (34) beinhaltet, das fest auf dem tragenden Körper (40) des Roboters (400) montiert ist und unten an dem Schubelement (32) befestigt ist und betätigt werden kann, um die vertikale Hin- und Herbewegung des Schubelementes (32) zu bewirken;
und dadurch, dass das Schubelement (32) so auf dem Schieberorgan (34) montiert ist, dass sich die obere Anschlagfläche (320) vertikal unterhalb der Linie von Greifzangen (30) befindet.

6. Vorrichtung nach dem vorhergehenden Anspruch, worin die Entnahmeeinrichtung (5) zumindest eine archimedische Schraube oder Schnecke (50) beinhaltet, die um eine mittlere Drehachse drehbar ist, die durch den Auslauf (U) der Maschine (M) führt, wobei ein erstes Ende der Schnecke (50) am Auslauf (U) der Maschine (M) angeordnet ist und die Schnecke an ihrem seitlichen Umfang eine spiralförmige Rille (500) aufweist, die um die mittlere Achse herum angeordnet ist, wobei diese Rille (500) so bemessen ist, dass sie einen vertikal ausgerichteten Artikel (1) aufnehmen kann, indem sie einen Anschlag für die Seitenwand (110) des Artikels (1) bildet, wobei die Rille (500) ferner am ersten Ende der Schnecke (50) beginnt, so dass sie die am Auslauf (U) der Maschine (M) dargebotenen Artikel (1) direkt aufnehmen kann.

7. Vorrichtung nach dem vorhergehenden Anspruch, **dadurch gekennzeichnet, dass** die Auflageführung (52) gerade ist, parallel zu der Drehachse der Schnecke (50) angeordnet werden kann und ferner relativ dazu so angeordnet werden kann, dass die Rille (500) der Schnecke (50) zusammen mit der Auflageführung (52) die mehreren beweglichen Aufnahmen (51) bildet, die in einer Reihe entlang der Längsausdehnung der Schnecke (50) angeordnet sind, so dass jeder Artikel (1) in jeder der beweglichen Aufnahmen (51) untergebracht werden kann, indem er in der Rille (500) aufgenommen wird und mit dem entsprechenden Halteelement (10) auf der geraden Führung (52) aufliegt, so dass die Unterseite (11) frei bleibt, wobei sich jede bewegliche Aufnahme (51) entlang der Längsausdehnung der Schnecke (50) vom Auslauf der Maschine (M) weg bewegt, wenn die Schnecke (50) in Drehung versetzt wird.

8. Vorrichtung nach dem vorhergehenden Anspruch, worin ein gerader Durchgang (520) in der Auffageführung (52) vorgesehen ist, wobei dieser gerade Durchgang (520) parallel zu der Symmetrieachse der Schnecke (50) verläuft und durch zwei einander zugewandte seitliche Kanten (521, 522) gebildet wird, wobei der gerade Durchgang (520) oberhalb der Schnecke (50) angeordnet ist und eine Breite aufweist, die derart bemessen ist, dass die Artikel (1) nur bis zu ihren jeweiligen Halteelementen (10) frei zwischen den seitlichen Kanten (521, 522) eingeführt werden, so dass die zwei gegenüberliegenden Seiten jedes Halteelements (10) jeweils aufliegend auf einer der verschiedenen seitlichen Kanten (521, 522) aufgenommen werden, die den Durchgang (50) des Führungs- und Auflageelementes (52) seitlich begrenzen und bilden.

9. Vorrichtung nach dem vorhergehenden Anspruch, anwendbar zum Überführen länglicher Artikel (1), die eine oder mehrere Seitenwände (110) beinhalten, wobei die Vorrichtung (A) **dadurch gekennzeichnet ist, dass** jede Greifzange (30) ein Paar Backen (31) beinhaltet, die wechselseitig relativ zu einer Drehachse drehbar sind, um sich abwechselnd einander anzunähern und somit die Greifzange (30) zu schließen, oder sich voneinander zu entfernen und somit die Greifzange (30) zu öffnen, wobei die Backen (31) derart geformt sind, dass sich die Greifzange (30) beim Schließen so mit einem Artikel (1) verbinden kann, dass die von den Backen (31) umschlossene Seitenwand (110) fest gegriffen wird.

10. Vorrichtung nach Anspruch 5 zum Überführen von Artikeln (1) zu Behältern, die jeweils ein Tray (20) für Artikel (1) aus zerbrechlichem Material beinhalten, welches wiederum mehrere offene Aufnahmefächer (210) beinhaltet, die von oben zugänglich sind und dazu dienen, die horizontal liegenden Artikel (1) einzeln aufzunehmen, so dass die Artikel (1) voneinander beabstandet sind, wobei die Vorrichtung (A) **dadurch gekennzeichnet** ist, das jede der Greifzangen (30) ein Paar Backen (31) beinhaltet, die so bemessen sind, dass sie in ein Aufnahmefach (210) eingeführt werden können, um einen Artikel (1) zu ergreifen; und dass ferner die Greifinittel eine Drehvorrichtung beinhalten, die mit den mehreren Greifzangen (30) verbunden ist, um die von der Entnahmeeinrichtung (5) aufgenommenen Artikel (1) so drehen zu können, dass sie horizontal angeordnet und somit in die entsprechenden Aufnahmefächer (210) des Trays (20) eingelegt werden können.

11. Vorrichtung nach einem der Ansprüche 5, 6 oder 9, anwendbar zum Überführen von Artikeln (1), die jeweils eine Spritze für Injektionen beinhalten, wobei diese Spritze einen Zylinder (100) zur Aufnahme der zu injizierenden Flüssigkeit beinhaltet, deren oberes Halteelement (10) aus einem vom Rand der Mündung des Zylinderröhrchens (100) gebildeten Bund besteht, und deren Unterseite (11) aus dem unteren Ende einer Schutzkappe besteht, in der die Nadel der Spritze untergebracht ist, wobei die Vorrichtung (A) **dadurch gekennzeichnet ist, dass** die Auflageführung (52), die Rille (500) der Schnecke (50), die Backen (31) der Greifzangen (30) und das Schubelement (32) derart bemessen sind, dass sie jeweils geeignet sind: gleitbar aufliegend die Bünde der Zylinder der Spritzen aufzunehmen, die Spritzen aufzunehmen, indem sie einen Anschlag für deren Seitenwand bildet, die Seitenwand der Spritze fest zu ergreifen und das untere Ende der Schutzkappe zu schieben.

12. Vorrichtung nach einem der Ansprüche 5, 6 oder 9, anwendbar zum Überführen von Artikeln (1), die jeweils eine Spritze für Injektionen und eine Schutzvorrichtung zum Aufnehmen und Festhalten der Nadel nach erfolgter Injektion beinhalten, wobei diese Schutzvorrichtung zumindest teilweise zwischen ihren Seitenwänden den Zylinder (100) der Spritze einschließt, seitliche Vorsprünge aufweist, welche das Halteelement (10) des Artikels bilden, und schließlich ein unteres Ende aufweist, das die Unterseite (11) bildet, wobei die Vorrichtung (A) **dadurch gekennzeichnet ist, dass** die Auffageführung (52), die Rille (500) der Schnecke (50), die Backen (31) der Greifzangen (30) und das Schubelement (32) derart bemessen sind, dass sie jeweils geeignet sind: gleitbar aufliegend die seitlichen Vorsprünge der Schutzvorrichtung aufzunehmen, die Schutzvorrichtung aufzunehmen, indem sie einen Anschlag für deren Seitenwand bildet, die Seitenwand der Schutzvorrichtung fest zu ergreifen und das untere Ende der Schutzvorrichtung zu schieben.

## Revendications

1. Une méthode pour transférer une pluralité d'articles (1), au moins partiellement réalisés dans un matériau fragile, par exemple des seringues pour injections, d'une machine automatique (M) qui les présente au niveau de sa sortie (U), séparés les uns des autres, à des contenants (2, 20) comprenant une pluralité de logements (21, 210) dans chacun desquels les articles peuvent être logés individuellement et séparément les uns des autres, où chacun des articles (1) comprend un élément de support (10) qui dépasse latéralement, et un fond (11) au niveau d'une extrémité inférieure de celui-ci, la méthode comprenant les phases suivantes :
a. disposer une pluralité d'articles (1) présentés à la sortie (U) de la machine automatique (M) dans une formation prédéfinie dans laquelle ils sont maintenus séparés les uns des autres ;
b. prélever plus d'un des articles (1) disposés durant la phase a. et, en les maintenant séparés les uns des autres, les amener à un contenant (2, 20) ; et
c. introduire les articles (1), en les maintenant séparés les uns des autres, dans le contenant (2, 20), et où
- durant la phase a., une sous-phase suivante est effectuée : disposer les articles (1), alors qu'ils se trouvent dans la formation prédéfinie, en appui sur les éléments de support (10) respectifs avec le fond (11) de ceux-ci laissé libre, et les sous-phases suivantes sont effectuées :
éloigner les articles (1) progressivement présentés à la sortie (U) de la machine (M) de ladite sortie (U), de manière à former au moins une file, comprenant une pluralité d'articles (1) ;
déplacer la file d'articles (1) ayant quitté la machine (M) pour l'éloigner de la sortie (U) de la machine (M), ladite file se trouvant dans la formation prédéfinie ;
déplacer la file d'articles (1) ayant quitté la machine (M) de façon continue ;
- durant la phase b., une sous-phase suivante est effectuée : pousser le fond (11) de plusieurs articles (1) vers le haut de manière à soulever les articles (1) tout en les maintenant en appui par le biais du fond (11) de ceux-ci, en les maintenant séparés, et de manière à ce qu'une partie supérieure (101) respective comprenant l'élément de support (10) soit soulevée et libre ; puis prendre les articles soulevés (1) par ladite partie supérieure (101) de ceux-ci, en les maintenant séparés les uns des autres, retirer les articles (1) de la file selon un mode de successivité, alors que ladite file se déplace, et les amener au contenant (2, 20).

2. La méthode selon la revendication précédente, utilisable pour transférer des articles (1) allongés, au moins partiellement réalisés dans un matériau fragile, dans des contenants comprenant chacun un plateau (20) pour contenir les articles (1) allongés, comprenant à son tour une pluralité de logements ouverts (210) pour loger séparément des articles (1) respectifs reposant à l'horizontale, les logements (210) étant disposés entre eux de manière à être séparés les uns des autres, la méthode étant **caractérisée en ce que** :
durant la phase a., une sous-phase suivante est effectuée : disposer chaque article (1) verticalement lorsqu'il se trouve dans la formation prédéfinie susmentionnée ; et **en ce que**, avant la phase c. ou en même temps que celle-ci, une phase suivante est effectuée : tourner chaque article (1) prélevé durant la phase b. de manière à disposer ledit article (1) à l'horizontale.

3. La méthode selon l'une quelconque des revendications précédentes, utilisable quand une ligne de transport (L) est prévue pour transporter les contenants (2, 20), en présentant des contenants (2, 20) sans articles (1) et en les éloignant dès lors que tous leurs logements (21, 210) logent des articles (1) respectifs, dans ces contenants (2, 20) les logements (21, 210) étant disposés en au moins une file, la méthode étant **caractérisée en ce que** :
durant la phase a., une sous-phase suivante est effectuée : disposer, dans la formation prédéfinie, un nombre d'articles (1) qui est au moins égal au nombre de logements (21, 210) de la file dans un contenant (2, 20) ;
durant la phase b., une sous-phase suivante est effectuée : prélever et amener au contenant (2, 20) un nombre d'articles (1) égal au nombre de logements (21, 210) de la file dans un contenant (2, 20),
durant la phase c., une sous-phase suivante est effectuée : introduire chacun des articles prélevés et amenés au contenant durant la phase b. dans un logement respectif de la file dans le contenant,
les phases a., b. et c. sont répétées en les appliquant cycliquement à chacun des logements (21, 210) et sont mises en oeuvre de manière à ce que la phase a. ait une durée qui est au plus égale à la somme d'une durée de la phase b. et de la phase c., de sorte que la phase a. d'un cycle durant laquelle sont prédisposés les articles destinés à être introduits dans une file donnée de logements (21, 210) d'un contenant (2, 20) puisse être exécutée durant les phases b. et c. appliquées durant le cycle précédent à une autre file de logements (21, 210) du même contenant (2, 20) ou d'un autre contenant ; et **en ce que**
une fois que des articles (1) respectifs sont logés dans tous les logements (21, 210) du contenant (2, 20), le contenant (2) est éloigné par le biais de la ligne de transport (L) ;
ce par quoi les logements (21, 210) d'autres contenants (2, 20) peuvent recevoir des articles (1) respectifs et les phases de la méthode peuvent être répétées du début, tant que des articles (1) sont présentés à la sortie de la machine (M).

4. La méthode selon la revendication 3, utilisable quand la ligne de transport (L) transporte les contenants (2, 20) de façon continue, où la phase c. est mise en oeuvre comme suit : introduire les articles (1) dans les logements (21, 210) respectifs d'une file dans un contenant (2, 20), en les maintenant séparés les uns des autres et selon un mode de successivité, alors que les contenants se déplacent portés par la ligne de transport (L).

5. Un appareil pour transférer une pluralité d'articles (1) allongés au moins partiellement réalisés dans un matériau fragile, comme par exemple des seringues pour injections, comprenant chacun une ou plusieurs parois latérales (110), un élément de support (10) qui dépasse latéralement, et un fond (11) au niveau d'une extrémité inférieure de celui-ci, par l'intermédiaire d'une machine automatique (M), qui présente les articles (1) à sa sortie (U), séparés les uns des autres, à des contenants (2, 20) comprenant une pluralité de logements (21, 210) dans chacun desquels les articles (1) peuvent être logés individuellement de manière à être séparés les uns des autres, lesdits contenants (2, 20) étant transportés par une ligne de transport (L) qui présente les contenants (2, 20) sans articles (1) et éloigne ces mêmes contenants dès lors que tous les logements (21, 210) de ceux-ci logent des articles (1) respectifs, dans lesdits contenants les logements étant disposés en au moins une file, l'appareil comprenant :
au moins un dispositif de prélèvement (5) pour recevoir et prendre livraison d'une pluralité d'articles (1) qui quittent la machine (M) au fur et à mesure qu'ils sont présentés à la sortie (U) de celle-ci, le dispositif de prélèvement (5) comprend au moins un guide d'appui (52) pour supporter les articles (1) présentés à la sortie (U) de la machine (M), le guide d'appui (52) étant configuré de manière à pouvoir recevoir en appui coulissant les éléments de support (10) des articles, à laisser un fond (11) de ceux-ci libre et à les éloigner de la sortie (U) de la machine (M), et une pluralité de logements mobiles (51) pour loger séparément des articles (1) respectifs présentés à la sortie (U) de la machine (M) et éloigner les articles logés (1) de la sortie (U) de la machine (M), les logements mobiles (51) étant disposés entre eux de manière à ce que les articles (1) qui y sont logés soient maintenus séparés les uns des autres,
au moins un dispositif de prise et de pose (3) pour retirer les articles du dispositif de prélèvement (5), les amener au contenant (2, 20) et les introduire dans des logements (21, 210) respectifs de celui-ci, le dispositif de prise et de pose (3) comprenant au moins des moyens de prise (30) configurés de manière à pouvoir prélever ou relâcher en alternance une pluralité d'articles (1) et à maintenir les articles prélevés séparés les uns des autres, les moyens de prise comprenant une pluralité de pinces (30), chacune desquelles pouvant se fermer pour saisir fermement un article (1) ou, en alternance, pouvant s'ouvrir pour relâcher ledit article, les pinces (30) étant disposées à une distance réciproque telle que les articles (1), durant leur prélèvement et une fois prélevés par une pince (30) respective, sont maintenus séparés les uns des autres,
l'appareil (A) étant **caractérisé en ce que** :
les moyens de prise (3) comprennent au moins un élément de poussée (32), pouvant être disposé inférieurement au dispositif de prélèvement et comprenant une surface supérieure de butée (320), l'élément de poussée (32) pouvant osciller verticalement de manière à ce que, lorsqu'il exécute une course vers le haut, il puisse pousser, avec sa surface supérieure (320), le fond libre (11) des articles logés dans les logements mobiles, entraînant ainsi une partie supérieure (101) des articles poussés (1) comprenant l'élément de support (10) à dépasser supérieurement du logement mobile et du guide d'appui (52), la pluralité de pinces (30) peut être disposée au-dessus du dispositif de prélèvement ; et **en ce que** chacune des pinces (30) respectives peut se fermer pour saisir un des articles (1) poussés vers le haut par l'élément de poussée (32), en le saisissant fermement par la partie supérieure (101) saillante de celui-ci, ou peut en alternance s'ouvrir pour relâcher l'article (1),
ledit dispositif de prise et de pose (3) comprend un robot (4) pour supporter et mouvoir la pluralité de pinces (30) dans l'espace, avec le robot (400) qui comprend :
un corps porteur (40) destiné à osciller entre des positions de chargement, sur un côté du dispositif de prélèvement, et une position de déchargement, à proximité d'un contenant transporté par la ligne de transport,
un guide (41) pour contraindre l'oscillation du corps porteur (40),
un moteur pour déplacer le corps porteur (40) le long du guide (41) ou pour arrêter le corps porteur (40) dans n'importe quelle position le long du guide (41) ; et
un bras de support (42) monté solidairement au corps porteur (40), sur ce bras de support (42) étant montée la pluralité de pinces (30), ledit bras de support (42) étant mobile pour déplacer les pinces (30) le long de deux axes cartésiens d'un plan vertical ;
le corps porteur (40), lorsqu'il se trouve dans la position de chargement, étant disposé par rapport au dispositif de prélèvement (5) de manière à ce que le bras de support (42) puisse soulever les pinces (30), quand ces mêmes pinces (30) ont saisi les articles (1), de manière à extraire les articles (1) des logements mobiles (51) respectifs, et le corps porteur (40), lorsqu'il se trouve dans la position de déchargement, étant en outre disposé par rapport à la ligne de transport (L) de manière à ce que le bras (42) puisse abaisser les pinces (30) portant les articles de manière à introduire lesdits articles (1) dans des logements respectifs d'un contenant auquel ils sont destinés, et **en ce que** les pinces (30) sont disposées sur une file horizontale ; le dispositif de prise et de pose (3) comprend en outre un organe pousseur (34) monté solidairement au corps porteur (40) du robot (400) et fixé inférieurement à l'élément de poussée (32), pouvant être activé pour causer l'oscillation verticale de l'élément de poussée (32) ; et **en ce que** l'élément de poussée (32) est monté sur l'organe pousseur (34) de manière à ce que la surface supérieure de butée (320) soit verticalement sous-jacente à la file de pinces (30).

6. L'appareil selon la revendication précédente, où le dispositif de prélèvement (5) comprend au moins une vis sans fin (50) pouvant tourner autour d'un axe de rotation central qui passe à travers la sortie (U) de la machine (M), une première extrémité de la vis sans fin (50) pouvant être positionnée au niveau de la sortie (U) de la machine (M), ladite vis sans fin ayant, au niveau d'une surface périphérique de celle-ci, une rainure hélicoïdale (500) disposée autour de l'axe central, ladite rainure (500) est dimensionnée de manière à pouvoir recevoir un article (1) disposé à la verticale, en venant en contact avec la paroi latérale (110) de l'article (1), la rainure (500) se développant en outre à partir de la première extrémité de la vis sans fin (50) de manière à pouvoir loger directement les articles (1) présentés à la sortie (U) de la machine (M).

7. L'appareil selon la revendication précédente, **caractérisé en ce que** le guide d'appui (52) est rectiligne, peut être positionné parallèlement à l'axe de rotation de la vis sans fin (50) et peut en outre être disposé par rapport à cette dernière de manière à ce que la rainure (500) de la vis sans fin (50) définisse, en association avec le guide d'appui (52), la pluralité de logements mobiles (51), qui sont disposés sur une file le long du développement longitudinal de la vis sans fin (50), chaque article (1) pouvant être logé dans chacun des logements mobiles (51), reçu dans la rainure (500) et en appui sur le guide rectiligne (52) par le biais de l'élément de support (10) respectif avec le fond (11) libre, chaque logement mobile (51) se déplaçant le long du développement longitudinal de la vis sans fin (50), en s'éloignant de la sortie de la machine (M) quand la vis sans fin (50) est mise en rotation.

8. L'appareil selon la revendication précédente, où un passage rectiligne (520) est prévu dans le guide d'appui (52), ledit passage rectiligne (520) est parallèle à l'axe de symétrie de la vis sans fin (50) et est défini par deux bords latéraux (521, 522) qui se font face, le passage rectiligne (520) étant disposé au-dessus de la vis sans fin (50) et ayant une largeur telle que les articles (1) peuvent être librement introduits entre les bords latéraux (521, 522) seulement jusqu'aux éléments de support (10) respectifs de ceux-ci, de sorte que deux côtés opposés de chaque élément de support (10) sont chacun reçu en appui sur un bord latéral (521, 522) différent flanquant et définissant le passage (520) de l'élément de guidage et d'appui (52).

9. L'appareil selon la revendication précédente, utilisable pour transférer des articles (1) allongés comprenant une ou plusieurs parois latérales (110), l'appareil (A) étant **caractérisé en ce que** chaque pince (30) comprend une paire de mâchoires (31) pouvant réciproquement tourner par rapport à un axe de rotation pour, en alternance, se rapprocher et fermer ainsi les pinces (30) ou pour s'éloigner et ouvrir les pinces (30), les mâchoires (31) ayant une forme telle que lorsque les pinces (30) se ferment, elles peuvent y accoupler un article (1), en saisissant fermement la paroi latérale (110) qui est serrée entre les mâchoires (31).

10. L'appareil selon la revendication 5, pour transférer des articles (1) à des contenants comprenant chacun un plateau (20) pour des articles (1) en matériau fragile, comprenant à son tour une pluralité de logements ouverts (210), accessibles par le dessus, et destinés à loger séparément les articles (1) reposant à l'horizontale de manière à ce que ces mêmes articles (1) soient séparés les uns des autres, l'appareil (A) étant **caractérisé en ce que** chaque pince (30) comprend une paire de mâchoires (31) qui sont dimensionnées de manière à s'introduire dans un logement (210) pour saisir un article (1) ; et les moyens de prise comprennent en outre un dispositif de rotation relié à la pluralité de pinces (30) de manière à pouvoir tourner les articles (1) prélevés du dispositif de prélèvement (5) afin de les disposer horizontalement pour pouvoir les introduire dans les logements (210) correspondants du plateau (20).

11. L'appareil selon une des revendications 5, 6 ou 9, utilisable pour transférer des articles (1) qui comprennent chacun une seringue pour injections, telle seringue comprend un cylindre (100) pour le liquide à injecter, dont l'élément supérieur (10) est un collier formé au niveau du bord d'une ouverture du tube (100), et dont le fond (11) est une extrémité inférieure d'un bouchon de sécurité qui loge une aiguille de la seringue, l'appareil (A) étant **caractérisé en ce que** le guide d'appui (52), la rainure (500) de la vis sans fin (50), les mâchoires (31) des pinces (30) et l'élément de poussée (32) sont dimensionnés de manière à pouvoir respectivement: recevoir en appui coulissant les colliers des cylindres des seringues, loger les seringues en venant en contact avec une paroi latérale de celles-ci, saisir fermement la paroi latérale des seringues et pousser l'extrémité inférieure du bouchon de sécurité.

12. L'appareil selon une des revendications 5, 6 ou 9, utilisable pour transférer des articles (1) qui comprennent chacun une seringue pour injections et un dispositif de sécurité pour loger et retenir l'aiguille quand une injection a été faite, tel dispositif de sécurité englobe au moins partiellement le cylindre (100) de la seringue entre ses parois latérales, comprend des saillies latérales qui constituent l'élément de support (10) de l'article et comprend enfin une extrémité inférieure qui constitue le fond (11), l'appareil (A) étant **caractérisé en ce que** le guide d'appui (52), la rainure (500) de la vis sans fin (50), les mâchoires (31) des pinces (30) et l'élément de poussée (32) sont dimensionnés de manière à pouvoir respectivement : recevoir en appui coulissant les saillies latérales du dispositif de sécurité, loger le dispositif de sécurité en venant en contact avec une paroi latérale de celui-ci, saisir fermement la paroi latérale du dispositif de sécurité et pousser l'extrémité inférieure du dispositif de sécurité.
